(19) Europäisches Patentamt
European Patent Office
Office européen des brevets

(11) Veröffentlichungsnummer: **0 424 316 A2**

(12) **EUROPÄISCHE PATENTANMELDUNG**

(21) Anmeldenummer: **90810776.6**

(51) Int. Cl.5: **C07D 249/20**

(22) Anmeldetag: **10.10.90**

(30) Priorität: **19.10.89 CH 3800/89**

(43) Veröffentlichungstag der Anmeldung:
**24.04.91 Patentblatt 91/17**

(84) Benannte Vertragsstaaten:
**DE FR GB IT**

(71) Anmelder: **CIBA-GEIGY AG**
**Klybeckstrasse 141**
**CH-4002 Basel(CH)**

(72) Erfinder: **Higel, Ariane, Dr.**
**Rue des Perdrix 3**
**F-68170 Rixheim(FR)**
Erfinder: **Stäubli, Sebastian**
**Lanzenbergstrasse 17**
**CH-4312 Magden(CH)**
Erfinder: **Thibaut, Daniel**
**Rue de l'Altenbach 12**
**F-68730 Michelbach-Le-Bas(FR)**
Erfinder: **Horisberger, Hans**
**Gartenstrasse 109**
**CH-4132 Muttenz(CH)**

(54) **Reinigung von 2-Aryl-2H-benztriazolen.**

(57) Beschrieben wird ein Verfahren, bei dem eine Schmelze eines 2-Aryl-2H-benztriazols enthaltend färbende Verunreinigungen oder eine Lösung davon in einem organischen, apolaren Lösungsmittel durch Behandlung mit einem stark sauren Kationenaustauscher auf Kunstharzbasis gereinigt wird.

EP 0 424 316 A2

## REINIGUNG VON 2-ARYL-2H-BENZTRIAZOLEN

Die vorliegende Erfindung betrifft ein verbessertes Reinigungsverfahren für 2-Aryl-2H-benztriazole.

2-Aryl-2H-benztriazole werden seit langem als UV-Absorber in Kunststoffen, Lacken oder anderen Materialien eingesetzt. Bei der Herstellung fallen 2-Aryl-2H-benztriazole üblicherweise verfärbt an. Für die obigen Anwendungen wird eine hohe Reinheit der Produkte verlangt, insbesondere die Abwesenheit von gefärbten Verunreinigungen. Diese Produkte müssen daher in der Regel zusätzlich gereinigt werden, beispielsweise durch Kristallisation, Destillation oder durch Extraktion. Im allgemeinen ist eine Kombination dieser Methoden erforderlich, um den gewünschten Reinheitsgrad zu erreichen. Dies verursacht zusätzliche Verfahrensschritte und somit zusätzliche Energie-, Rohstoff und Entsorgungskosten.

Es wurde jetzt ein verbessertes Verfahren zur Entfernung von färbenden Verunreinigungen aus 2-Aryl-2H-benztriazolen gefunden. Das neue Verfahren beruht auf der Erkenntnis, dass ein ausgewählter Ionenaustauschertyp in einer apolaren Umgebung der ionenaustauschenden Gruppen als Adsorbens für die färbenden Verunreinigungen eingesetzt werden kann. Unter diesen Bedingungen verläuft die Entfernung der färbenden Verunreinigungen praktisch ohne Ionenaustausch. Die Desorption der Verunreinigungen nach der Reinigungsoperation kann anschliessend durch einen Polaritätswechsel der Umgebung der ionenaustauschenden Gruppen erfolgen, indem man den Ionenaustauscher mit einem polaren Lösungsmittel regeneriert.

Das neue Reinigungsverfahren zeichnet sich durch einen geringen Bedarf an Adsorbens aus. Ferner ist das Adsorbens mehrfach regenerierbar. Das verbrauchte Adsorbens kann vollständig verbrannt werden, so dass keine Deponieprobleme auftreten. Ausserdem kann das Reinigungsverfahren mit Lösungsmitteln durchgeführt werden, die auch bei der Herstellung des 2-Aryl-2H-benztriazols verwendet werden. Diese Lösungsmittel können in den Prozess zurückgeführt werden. Ein weiterer Vorteil ist die verbesserte Qualität des gereinigten Produktes, so dass eine direkte Isolierung ohne zwischengeschaltete Kristalli sationsstufen möglich ist. Insgesamt wird die Umweltbelastung durch das neue Reinigungsverfahren erheblich reduziert.

Die vorliegende Erfindung betrifft ein Verfahren zur Reinigung von 2-Aryl-2H-benztriazolen, die färbende Verunreinigungen enthalten. Das Verfahren ist dadurch gekennzeichnet, dass man eine Schmelze eines 2-Aryl-2H-benztriazols oder eine Lösung davon in einem organischen, apolaren Lösungsmittel mit einem Kationenaustauscher auf Kunstharzbasis behandelt, der stark saure Gruppen enthält, welche wenigstens zum Teil in protonierter Form vorliegen.

Bei den erfindungsgemäss eingesetzten 2-Aryl-2H-benztriazolen kann es sich ganz allgemein um unsubstituierte oder um substituierte 2H-Benztriazole mit einem Arylrest, insbesondere einem Phenylrest, in 2-Position handeln. Vorzugsweise handelt es sich um unsubstituierte oder um substituierte 2-(2-Hydroxyphenyl)-2H-benztriazole, insbesondere um Verbindungen der Formel I

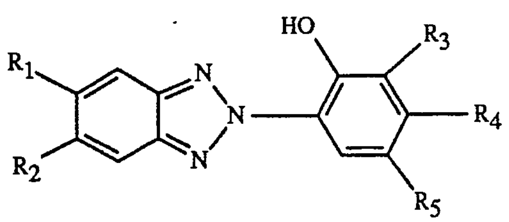 (I),

worin $R_1$ Wasserstoff, Chlor oder $C_1$-$C_{12}$ Alkyl ist, $R_2$ Wasserstoff, Chlor, $C_1$-$C_{12}$ Alkyl, $C_1$-$C_4$ Alkoxy, $C_2$-$C_9$ Alkoxycarbonyl, $C_7$-$C_9$ Phenylalkyl, Carboxyl oder $-SO_3H$ ist, $R_3$ Wasserstoff, Chlor, $C_1$-$C_{12}$ Alkyl, Cycloalkyl mit 5 oder 6 Ringkohlenstoffatomen, Phenyl, mit $C_1$-$C_8$Alkyl substituiertes Phenyl oder $C_7$-$C_9$ Phenylalkyl bedeutet, $R_4$ Wasserstoff, Chlor, $C_1$-$C_4$ Alkyl, $C_1$-$C_4$ Alkoxy oder Hydroxyl bedeutet, und $R_5$ $C_1$-$C_{12}$ Alkyl, $C_1$-$C_4$ Alkoxy, Cycloalkyl mit 5 oder 6 Ringkohlenstoffatomen, Phenyl, mit $C_1$-$C_8$ Alkyl substituiertes Phenyl, Chlor, $C_7$-$C_9$ Phenylalkyl oder $-C_nH_{2n}-COOR_6$ bedeutet, n für eine ganze Zahl von 0 bis 4 steht und $R_6$ Wasserstoff oder $C_1$-$C_{12}$ Alkyl bedeutet.

Solche Verbindungen sind beispielsweise in der US-A-4,041,044 oder in der EP-A-57,160 beschrieben. 2-Aryl-2H-benztriazole lassen sich beispielsweise durch reduktive Cyclisierung von o-Nitroazobenzolen herstellen, welche ihrerseits durch Diazotierung von o-Nitroanilinen und Umsetzung mit geeigneten Kupplungskomponenten erhältlich sind. Beispiele für solche Reaktionen sind in den US-A-4,041,044, EP-A-57,160, GB-A-1,494,824, GB-A-1,494,825 und GB-A-1,494,826 beschrieben. Der Inhalt der Beschreibungen dieser Dokumente ist ebenfalls Inhalt der vorliegenden Beschreibung.

Bevorzugt werden Verbindungen der Formel I gereinigt, worin $R_1$ Wasserstoff, Chlor oder $C_1$-$C_{12}$ Alkyl, insbesondere Wasserstoff oder Chlor ist, $R_2$ Wasserstoff bedeutet, $R_3$ Wasserstoff, $C_1$-$C_{12}$ Alkyl oder Phenyl-$C_1$-$C_3$-alkyl, insbesondere $\alpha,\alpha$-Dimethylbenzyl, ist, $R_4$ Wasserstoff bedeutet, $R_5$ $C_1$-$C_{12}$ Alkyl, insbesondere $C_1$-$C_8$ Alkyl, Phenyl-$C_1$-$C_3$-alkyl, insbesondere $\alpha,\alpha$-Dimethylbenzyl, oder eine Gruppe -$C_2H_4$-$COOR_6$ ist, und $R_6$ Wasserstoff oder $C_1$-$C_{12}$ Alkyl, insbesondere $C_1$-$C_8$ Alkyl, und ganz besonders Wasserstoff oder $C_1$-$C_5$ Alkyl bedeutet.

Ganz besonders bevorzugt werden Verbindungen der Formel I gereinigt, worin $R_1$ Wasserstoff oder Chlor ist, $R_2$ Wasserstoff bedeutet, $R_3$ Wasserstoff oder $C_1$-$C_8$ Alkyl ist, $R_4$ Wasserstoff bedeutet und $R_5$ $C_1$-$C_8$ Alkyl ist.

Die Natur der färbenden Verunreinigungen ist nicht bekannt. Diese liegen im allgemeinen in geringen Mengen im Rohprodukt vor, beispielsweise zu weniger als 1 Gew. %, bezogen auf das Rohprodukt. Die Entfernung dieser Verunreinigungen aus der eingesetzten Lösung bzw. Schmelze kann mit Routineverfahren überwacht werden, beispielsweise durch Beobachtung der Transmission der zu reinigenden bzw. der gereinigten Lösung bzw. Schmelze bei einer ausgewählten Wellenlänge.

Die im erfindungsgemässen Verfahren verwendeten Kationenaustauscher auf Kunstharzbasis müssen stark saure Gruppen enthalten und diese Gruppen müssen wenigstens zum Teil in protonierter Form vorliegen.

Beispiele für stark saure Gruppen sind Sulfonsäuregruppen oder Austauschergruppen mit einer vergleichbaren oder stärkeren Acidität. Vorzugsweise handelt es sich bei den stark sauren Gruppen um Sulfonsäuregruppen.

Die Adsorptionswirkung des verwendeten Kationenaustauschers hängt unter anderem von der Menge der dem zu reinigenden Material zugänglichen protonierten Austauschergruppen ab. Man wählt daher vorteilhaft Kationenaustauscher mit einer grossen Oberfläche und mit einem hohen Protonierungsgrad der Austauschergruppen. Ueblicherweise sollte die zugängliche Oberfläche der eingesetzten Kationenaustauscher mehr als 40 $m^2/g$ betragen (gemessen nach dem BET Verfahren).

Bevorzugt werden stark saure, makroporöse Kationenaustauscher auf Kunstharzbasis.

Der Begriff "makroporös" ist dem Fachmann bekannt und bezeichnet ein Kunstharz, dessen Poren bereits im Harz in ungequollenem Zustand enthalten sind und nicht erst bzw. nicht ausschliesslich durch Quellung entstehen. Diese Ionenaustauscher besitzen eine grosse Oberfläche, in der Regel mehr als 40 $m^2/g$, gemessen nach dem BET Verfahren, und sie sind porös. Die mittlere Porengrösse ist im allgemeinen kleiner als 7000 nm. Vorzugsweise ist die mittlere Porengrösse 2000 bis 7000 nm.

Man kann aber auch Kationenaustauscher in feinverteilter Form einsetzen, um eine grosse Oberfläche zu erzielen.

Besonders bevorzugt werden sulfonsäuregruppenhaltige, makroporöse Kationenaustauscher auf Basis von Polystyrol, die mit einem zum Aufbau der makrocyclischen Struktur ausreichenden Anteil von Divinylbenzol vernetzt sind.

Beispiele für solche makroporösen Ionenaustauscher sind die Produkte Lewatite® 2641, Lewatite® 2661 und Lewatite® 2611 der Fa. Bayer, Amberlyst® 15, Amberlyst® N-1010 und Amberlite® 200 der Fa. Rohm & Haas und Dowex® MSC-1 der Fa. Dow Chemicals.

Die Austauscherharze können in beliebiger Form eingesetzt werden, beispielsweise in Form von Membranen, Fasern oder insbesondere in gekörnter Form.

Die Säuregruppen des Kationenaustauschers müssen zumindest teilweise in protonierter Form vorliegen. Der für den Einzelfall ausreichende Anteil an protonierten Säuregruppen kann vom Fachmann durch Routinetests ermittelt werden, da die Reinigungswirkung mit der Anzahl der zugänglichen protonierten Gruppen zunimmt.

Vorzugsweise liegen praktisch die gesamten Säuregruppen des Kationenaustauschers in protonierter Form vor.

Die Reinigungswirkung wird unter anderem auch von der Polarität des verwendeten Lösungsmittels beeinflusst. Versuche haben gezeigt, dass mit polaren Lösungsmitteln, beispielsweise mit Alkoholen, keine befriedigende Reinigungswirkung erzielt wird.

Das apolare, organische Lösungsmittel darf keine Gruppen enthalten, die einen Ionenaus tausch mit den protonierten sauren Gruppen im Kationenaustauscher bewirken und es sollte üblicherweise eine Polarität $E_T^N$ von weniger als 0,30 aufweisen. $E_T^N$ ist ein empirischer Lösungsmittelparameter, der von der Solvatochromie eines Pyridinium-N-phenoxid-betains abgeleitet wird. Einzelheiten dazu sind von Ch. Reichert in "Solvents and Solvent Effects in Organic Chemistry", S. 407-411, Verlagsgesellschaft VCh (1988) beschrieben.

Beispiele für geeignete organische, apolare Lösungsmittel sind bei Raumtemperatur flüssige aliphatische oder aromatische Kohlenwasserstoffe, die gegebenenfalls halogeniert sein können, oder aliphatische

Ether.

Beispiele für geeignete aliphatische Kohlenwasserstoffe sind n-Hexan, n-Octan, Petrolether, Cyclohexan oder Methylcyclohexan.

Beispiele für geeignete aromatische Kohlenwasserstoffe sind Benzol, Toluol, Xylol oder Mesitylen.

Beispiele für geeignete halogenierte Kohlenwasserstoffe sind Dichlormethan, Chloroform, Trichlorethan, Tetrachlorethan, Chlorbenzol oder Dichlorbenzol.

Beispiele für geeignete aliphatische Ether sind Diethylether, Dibutylether, Tetrahydrofuran oder Dioxan.

Man kann auch Gemische von apolaren, organischen Lösungsmitteln einsetzen.

Besonders bevorzugt verwendet man bei Raumtemperatur flüssige aromatische Kohlenwasserstoffe, insbesondere Toluol oder Xylol.

Das zu reinigende 2-Aryl-2H-benztriazol kann in einem apolaren, organischen Lösungsmittel gelöst oder als Schmelze vorgelegt werden.

Die zu reinigende Lösung bzw. Schmelze kann, je nach Herstellungsverfahren des 2-Aryl-2H-benztriazols, neben dem allfälligen Lösungsmittel und den färbenden Verunreinigungen noch feste Verunreinigungen, insbesondere nicht oder nur teilweise gelöste Salze, wie Natriumsulfat, enthalten. Bei der Entfernung der färbenden Verunreinigungen können Salze stören, da ein Ionenaustausch stattfindet, und somit einerseits die zu reinigende Lösung bzw. Schmelze angesäuert wird und andererseits die protonierte Form der Säurereste im Kationenaustauscher verbraucht wird, was in der Regel zu einem Absinken der Adsorptionskapazität oder sogar zu einem Verschwinden des Reinigungseffektes führt. Die Adsorption der färbenden Verunreinigungen an sich erfolgt praktisch ohne einen nennenswerten Ionenaustausch.

In einer besonderen Ausführungsform des erfindungsgemässen Verfahrens wird eine Lösung enthaltend das verunreinigte 2-Aryl-2H-benztriazol in einem organischen, apolaren Lösungsmittel vor der Behandlung mit dem Kationenaustauscher klärfiltriert, um feste oder teilweise gelöste Salze daraus zu entfernen.

Dazu behandelt man zweckmässigerweise die Ausgangslösung in an sich bekannter Weise mit einem Filtrierhilfsmittel, beispielsweise mit Cellulose oder insbesondere mit Aktivkohle, und filtriert die Lösung.

Das erfindungsgmässe Verfahren kann ansatzweise oder vorzugsweise kontinuierlich ausgeführt werden.

Verfahrensparameter, wie Menge des Kationenaustauschers, Behandlungsdauer der verunreinigten Lösung bzw. Schmelze mit dem Kationenaustauscher und Temperatur der zu reinigenden Lösung bzw. Schmelze sind im wesentlichen nicht kritisch für die Durchführung des erfindungsgemässen Verfahrens. Diese Parameter werden so gewählt, dass der gewünschte Reinigungsgrad erzielt wird. Die Optimierung dieser Parameter ist dem Fachmann an sich bekannt. Die obere Temperaturgrenze für das Verfahren ist im allgemeinen durch die Zersetzungstemperatur des Kationenaustauschers gegeben, während die untere Temperaturgrenze im allgemeinen durch die Erstarrungstemperatur der Schmelze bzw. der einzusetzenden Lösung vorgegeben ist.

Die Reinigung kann drucklos oder unter Anwendung von Druck erfolgen. Vorzugsweise arbeitet man bei Atmosphärendruck.

Bei der ansatzweisen Verfahrensführung wird die zu reinigende Schmelze oder Lösung üblicherweise in ein Gefäss gegeben, das den Kationenaustauscher enthält. Soll eine Lösung gereinigt werden, so wird der Kationenaustauscher in der Regel bereits zusammen mit einem organischen, apolaren Lösungsmittel vorgelegt, vorzugsweise mit demjenigen Lösungsmittel, das auch in der zu reinigenden Lösung enthalten ist. Nachdem der gewünschte Reinigungsgrad der Lösung erzielt ist, wird der Kationenaustauscher entfernt, beispielsweise durch Filtration.

Bei der kontinuierlichen Verfahrensführung wird der Kationenaustauscher üblicherweise in einer Austauschersäule vorgelegt. In der Regel enthält die Säule neben dem Kationenaustauscher bereits ein organisches, apolares Lösungsmittel, vorzugsweise dasjenige Lösungsmittel, das auch in der zu reinigenden Lösung enthalten ist. Man kann auch mehrere Austauschersäulen hintereinander schalten.

In einer bevorzugten Ausführungsform des erfindungsgemässen Verfahrens wird die Schmelze oder die Lösung enthaltend das verunreinigte 2-Aryl-2H-benztriazol durch mindestens eine Austauschersäule, insbesondere durch zwei hintereinander geschaltetete Austauschersäulen geleitet, die den Kationenaustauscher in für den Kolonnenbetrieb geeigneter Form, beispielsweise in gekörnter Form, wie in der Form von Kugeln oder von Pellets, enthalten.

In einer ganz besonders bevorzugten Ausführungsform des erfindungsgemässen Verfahrens werden zwei Austauschersäulen hintereinander geschaltet und das aus der ersten Säule austretende Eluat wird beobachtet, beispielsweise durch Verfolgen der Transmission der Lösung bei einer ausgewählten Wellenlänge. Ist die Reinigungskapazität der ersten Säule erschöpft, so wird diese entfernt und der Regenerierung zugeführt. Vor die ursprüngliche zweite Säule kann dann praktisch ohne Unterbrechung der Reinigungsoperation eine frische Austauschersäule geschaltet werden oder die ursprüngliche zweite Säule wird jetzt als

erste Säule benutzt und eine frische zweite Säule wird zugeschaltet.

Der Durchsatz durch eine Austauschersäule beträgt im allgemeinen 0,5 bis 30 Bettvolumina/h, insbesondere 1 bis 6 Bettvolumina/h.

Das gereinigte 2-Aryl-2H-benztriazol wird üblicherweise durch Eindampfen des Lösungsmittels isoliert. Eine weitere Reinigung, beispielsweise durch Umkristallisieren oder durch Destillation, ist im allgemeinen nicht mehr nötig. Erforderlichenfalls wird das anfallende Produkt noch zerkleinert, beispielsweise durch Vermahlen.

In einer besonders bevorzugten Variante des erfindungsgemässen Verfahrens erfolgt das Eindampfen des Lösungsmittels in einem Wirbelschichtgranulator, einem Zerstäubungstrockner oder einer Kombination beider Apparate. In dieser Ausführungsform fällt das Produkt direkt in gebrauchsfertiger, rieselfähiger und staubfreier Form an.

Die Regenerierung des verbrauchten Austauschers kann auf einfache Art und Weise durch einen Polaritätswechsel des Lösungsmittels erfolgen. Die Massnahmen bei der Regenerierung richten sich im wesentlichen danach, ob der verbrauchte Ionenaustauscher im wesentlichen noch in protonierter Form vorliegt, und somit durch eine blosse Desorption der Verunreinigungen regeneriert werden soll; oder ob an dem überwiegenden Anteil der Säuregruppen ein Ionenaustausch stattgefunden hat, beispielsweise infolge der Anwesenheit von Salzen in der zu reinigenden Lösung, und daher die blosse Desorption der Verunreinigungen nicht ausreicht, um in der folgenden Adsorptionsstufe eine ausreichende Reinigungswirkung zu ergeben.

Die Regenerierung des im wesentlichen in protonierter Form vorliegenden Kationenaustauschers umfasst die folgenden Schritte:

i) Spülen des beladenen Kationenaustauschers mit einem organischen, apolaren Lösungsmittel, bis praktisch das gesamte anhaftende 2-Aryl-2H-benztriazol entfernt ist,

ii) Desorption der Verunreinigungen auf dem Kationenaustauscher durch Behandlung mit einem polaren Lösungsmittel oder mit einem Gemisch enthaltend ein apolares und ein polares Lösungsmittel, insbesondere mit einem organischen, polaren Lösungsmittel oder mit einem Gemisch enthaltend ein organisches, apolares Lösungsmittel und ein organisches, polares Lösungsmittel, in dem die adsorbierten Verbindungen ausreichend löslich sind, und

iii) Verdrängung des polaren Lösungsmittels mit einem organischen, apolaren Lösungsmittel.

Die Regenerierung des im wesentlichen in nicht protonierter Form vorliegenden Kationenaustauschers umfasst folgende Schritte:

iv) Spülen des beladenen Kationenaustauschers mit einem organischen, apolaren Lösungsmittel, bis praktisch das gesamte anhaftende 2-Aryl-2H-benztriazol entfernt ist,

v) Desorption der Verunreinigungen auf dem Kationenaustauscher durch Behandlung mit einem polaren Lösungsmittel, oder mit einem Gemisch enthaltend ein apolares und ein polares Lösungsmittel, insbesondere mit einem organischen, polaren Lösungsmittel oder mit einem Gemisch enthaltend ein organisches, apolares Lösungsmittel und ein organisches, polares Lösungsmittel, in dem die adsorbierten Verbindungen ausreichend löslich sind,

vi) Regenerierung der protonierten Form des Kationenaustauschers durch Behandlung mit einer Säure, und

vii) Verdrängung des polaren Lösungsmittels und der Säure mit einem organischen, apolaren Lösungsmittel.

Schritte v) und vi) können auch gleichzeitig durchgeführt werden.

Als polares Lösungsmittel in der Desorptionsstufe eignen sich beispielsweise Alkohole, Phenole oder Carbonsäuren.

Besonders bevorzugt verwendet man Alkohole, insbesondere einwertige aliphatische Alkohole, wie Methanol, Ethanol, Propanol, Butanol, Pentanol oder Hexanol.

Ganz besonders bevorzugt verwendet man in der Desorptionsstufe ein Gemisch aus dem organischen, apolaren Lösungsmittel, das in der Adsorptionsstufe verwendet wurde, und aus einem organischen und polaren Lösungsmittel.

Soll die nicht protonierte Form des Kationenaustauschers regeneriert werden, so verwendet man vorzugsweise einwertige aliphatische Alkohole, die eine Sulfonsäure, beispielsweise p-Toluolsulfonsäure gelöst enthalten.

In einer ganz besonders bevorzugten Variante des erfindungsgemässen Verfahrens wird für die zu reinigende Lösung des 2-Aryl-2H-benztriazols und in den Schritten i), iii), iv) und vii) und gegebenenfalls in den Schritten ii) und v) als organisches, apolares Lösungsmittel ein aromatischer Kohlenwasserstoff, insbesondere Xylol oder Toluol, verwendet, und in den Schritten ii) und v) wird als polares Lösungsmittel ein einwertiger aliphatischer Alkohol, insbesondere Butanol, verwendet. Ganz besonders bevorzugt verwendet man in den Schritten ii) und v) ein Gemisch aus Xylol und 2-Butanol.

Die folgenden Beispiele erläutern die Erfindung.

Beispiel 1 : Reinigung von

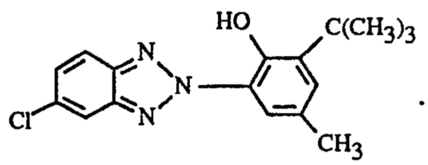

Eine stark dunkel gefärbte Lösung von 40 Gew.%, bezogen auf die Lösung, an 2-(2-Hydroxy-3-tert.butyl-5-methylphenyl)-2H-5-chlorbenztriazol in Xylol (Menge an färbenden Verunreinigungen< 1 Gew.%) wird bei 85 °C über Aktivkohle klärfiltriert und anschliessend über zwei in Reihe geschaltete Austauschersäulen enthaltend Amberlyst® 15 geleitet. Der Durchsatz der Lösung durch eine Säule beträgt 2 Bettvolumina/h. Die aus der zweiten Säule austretende Lösung ist praktisch farblos.

Die Qualität der gereinigten Lösung wird jeweils am Ausgang der ersten und der zweiten Säule überwacht. Durch die zunehmende Beladung der ersten Säule ergibt sich im Verlaufe der Reinigung eine Qualitätsabnahme, welche von der zweiten Säule aufgehoben wird. Nachdem die erste Säule keine Reinigungswirkung mehr zeigt, wird sie ausgetauscht und der beladene Ionenaustauscher der Regeneration zugeführt.

Beispiel 2 : Regenerierung des beladenen Ionenaustauschers

Der gemäss Beispiel 1 beladene Ionenaustauscher wird in einem dreistufigen Prozess regeneriert. Zunächst wird die Säule bei 85 °C mit Xylol gespült, um das anhaftende 2-Hydroxyaryl-2H-benztriazol zu entfernen. Anschliessend erfolgt die Desorption der färbenden Verunreinigungen bei 70 °C durch Eluieren der Säule mit einem Gemisch von Xylol und 2-Butanol (20 : 80 Volumenteile). Nachdem das Eluat praktisch keine Verfärbung mehr erkennen lässt, wird die Säule mit Xylol bei 70 °C gespült, um das polare Lösungsmittel zu verdrängen. Anschliessend kann die Säule wieder im Reinigungsverfahren eingesetzt werden.

Beispiel 3 : Reinigung von

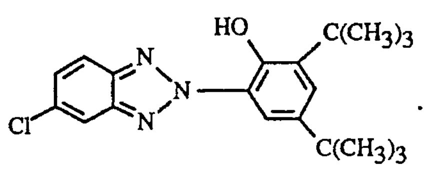

Eine stark dunkel gefärbte Lösung von 50 Gew.%, bezogen auf die Lösung, an 2-(2-Hydroxy-3,5-di-tert.butyl-phenyl)-2H-5-chlorbenztriazol in Xylol wird in Analogie zu Beispiel 1 klärfiltriert und über zwei in Reihe geschaltete Austauschersäulen enthaltend Amberlyst® 15 gereinigt. der Durchsatz der Lösung durch eine Säule beträgt 2 Bettvolumina/h. Die aus der zweiten Säule austretende Lösung ist praktisch farblos.

Beispiel 4: Reinigung von

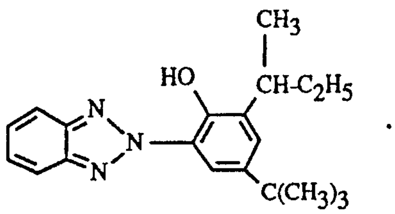

Eine stark dunkel gefärbte Lösung von 50 Gew.%, bezogen auf die Lösung, an 2-(2-Hydroxy3-isobutyl-5-tert.butyl-phenyl)-2H-benztriazol in Xylol wird in Analogie zu Beispiel 1 klärfiltriert und über zwei in Reihe geschaltete Austauschersäulen enthaltend Amberlyst® 15 gereinigt. Der Durchsatz der Lösung durch eine Säule beträgt 1 Bettvolumen/h. Die aus der zweiten Säule austretende Lösung ist praktisch farblos.

Beispiel 5 : Reinigung von

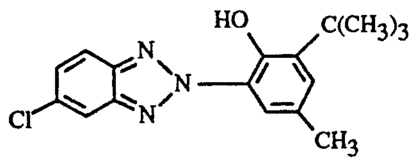

ohne vorherige Klärfiltration.

Man arbeitet in Analogie zu Beispiel 1 ohne Vorschaltung einer Klärfiltration mit Aktivkohle. Nach dem Passieren der Austauschersäulen ist die ursprünglich stark dunkel gefärbte Lösung von 2-(2-Hydroxy-3-tert.butyl-5-methylphenyl)-2H-5-chlorbenztriazol praktisch farblos.

Beispiel 6 : Reinigung von

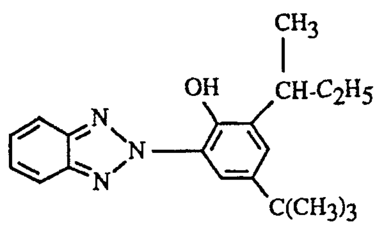

als Schmelze.

Man arbeitet bei 90° C in Analogie zu Beispiel 4 mit oder ohne Vorschaltung einer Klärfiltration mit Aktivkohle. Nach dem Passieren der Austauschersäulen ist die ursprünglich stark dunkel gefärbte Schmelze von 2-(2-Hydroxy-3-isobutyl-5-tert.butyl-phenyl)-2H-benztriazol entfärbt.

## Ansprüche

1. Verfahren zur Reinigung von 2-Aryl-2H-benztriazolen, die färbende Verunreinigungen enthalten, dadurch gekennzeichnet, dass man eine Schmelze eines 2-Aryl-2H-benztriazols oder eine Lösung davon in einem organischen, apolaren Lösungsmittel mit einem Kationenaustauscher auf Kunstharzbasis behandelt, der stark saure Gruppen enthält, welche wenigstens zum Teil in protonierter Form vorliegen.
2. Verfahren gemäss Anspruch 1, dadurch gekennzeichnet, dass es sich bei dem 2-Aryl-2H-benztriazol um eine Verbindung der Formel I handelt

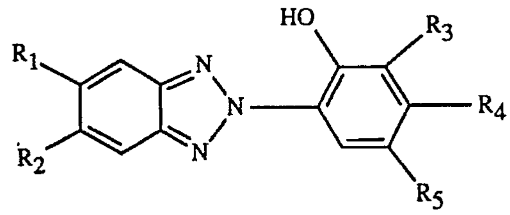

(I),

worin $R_1$ Wasserstoff, Chlor oder $C_1$-$C_{12}$ Alkyl ist, $R_2$ Wasserstoff, Chlor, $C_1$-$C_{12}$ Alkyl, $C_1$-$C_4$ Alkoxy, $C_2$-$C_9$ Alkoxycarbonyl, $C_7$-$C_9$ Phenylalkyl, Carboxyl oder $-SO_3H$ ist, $R_3$ Wasserstoff, Chlor, $C_1$-$C_{12}$ Alkyl, Cycloalkyl mit 5 oder 6 Ringkohlenstoffatomen, Phenyl, mit $C_1$-$C_8$Alkyl substituiertes Phenyl oder $C_7$-$C_9$ Phenylalkyl bedeutet, $R_4$ Wasserstoff, Chlor, $C_1$-$C_4$ Alkyl, $C_1$-$C_4$ Alkoxy oder Hydroxyl bedeutet, und $R_5$ $C_1$-$C_{12}$ Alkyl, $C_1$-$C_4$ Alkoxy, Cycloalkyl mit 5 oder 6 Ringkohlenstoffatomen, Phenyl, mit $C_1$-$C_8$ Alkyl substituiertes Phenyl, Chlor, $C_7$-$C_9$ Phenylalkyl oder $-C_nH_{2n}-COOR_6$ bedeutet, n für eine ganze Zahl von 0 bis 4 steht und $R_6$ Wasserstoff oder $C_1$-$C_{12}$ Alkyl bedeutet.

3. Verfahren gemäss Anspruch 1, dadurch gekennzeichnet, dass es sich bei den stark sauren Gruppen um Sulfonsäuregruppen handelt.

4. Verfahren gemäss Anspruch 1, dadurch gekennzeichnet, dass es sich bei dem Kationenaustauscher um einen stark sauren, makroporösen Kationenaustauscher auf Kunstharzbasis handelt.

5. Verfahren gemäss Anspruch 4, dadurch gekennzeichnet, dass ein sulfonsäuregruppenhaltiger, makroporöser Kationenaustauscher auf Basis von Polystyrol eingesetzt wird, der mit einem zum Aufbau der makrocyclischen Struktur ausreichenden Anteil von Divinylbenzol vernetzt ist.

6. Verfahren gemäss Anspruch 1, dadurch gekennzeichnet, dass als organisches, apolares Lösungsmittel ein bei Raumtemperatur flüssiger aromatischer Kohlenwasserstoff, insbesondere Toluol oder Xylol, verwendet wird.

7. Verfahren gemäss Anspruch 1, dadurch gekennzeichnet, dass eine Lösung enthaltend das verunreinigte 2-Aryl-2H-benztriazol in einem organischen, apolaren Lösungsmittel vor der Behandlung mit dem Kationenaustauscher klärfiltriert wird, um feste oder teilweise gelöste Salze daraus zu entfernen.

8. Verfahren gemäss Anspruch 7, dadurch gekennzeichnet, dass die Klärfiltration durch Behandlung der Lösung mit Aktivkohle erfolgt.

9. Verfahren gemäss Anspruch 1, dadurch gekennzeichnet, dass die Schmelze oder die Lösung enthaltend das verunreinigte 2-Aryl-2H-benztriazol durch mindestens eine Austauschersäule, insbesondere durch zwei hintereinander geschaltetete Austauschersäulen, geleitet wird, die den Kationenaustauscher in gekörnter Form enthalten.

10. Verfahren gemäss Anspruch 9, dadurch gekennzeichnet, dass der Durchsatz durch eine Austauschersäule 1 bis 6 Bettvolumina/h beträgt.

11. Verfahren gemäss Anspruch 1, dadurch gekennzeichnet, dass die Lösung des 2-Aryl-2H-benztriazols in einem apolaren organischen Lösungsmittel nach der Reinigung durch den Kationenaustauscher durch Eindampfen des Lösungsmittels isoliert wird.

12. Verfahren gemäss Anspruch 11, dadurch gekennzeichnet, dass das Eindampfen des Lösungsmittels in einem Wirbelschichtgranulator, einem Zerstäubungstrockner oder einer Kombination beider Apparate erfolgt.

13. Verfahren gemäss Anspruch 1, dadurch gekennzeichnet, dass der im wesentlichen in protonierter Form vorliegende Kationenaustauscher nach der Erschöpfung der Adsorptionskapazität regeneriert wird, wobei folgende Schritte durchlaufen werden:

i) Spülen des beladenen Kationenaustauschers mit einem organischen, apolaren Lösungsmittel, bis praktisch das gesamte anhaftende 2-Aryl-2H-benztriazol entfernt ist,

ii) Desorption der Verunreinigungen auf dem Kationenaustauscher durch Behandlung mit einem polaren Lösungsmittel oder mit einem Gemisch enthaltend ein apolares und ein polares Lösungsmittel, in dem die adsorbierten Verbindungen ausreichend löslich sind, und

iii) Verdrängung des polaren Lösungsmittels mit einem organischen, apolaren Lösungsmittel.

14. Verfahren gemäss Anspruch 1, dadurch gekennzeichnet, dass der im wesentlichen in nicht protonierter Form vorliegende Kationenaustauscher nach der Erschöpfung der Adsorptionskapazität regeneriert wird, wobei folgende Schritte durchlaufen werden:

iv) Spülen des beladenen Kationenaustauschers mit einem organischen, apolaren Lösungsmittel, bis praktisch das gesamte anhaftende 2-Aryl-2H-benztriazol entfernt ist,

v) Desorption der Verunreinigungen auf dem Kationenaustauscher durch Behandlung mit einem polaren Lösungsmittel, oder mit einem Gemisch enthaltend ein apolares und ein polares Lösungsmittel, in dem

die adsorbierten Verbindungen ausreichend löslich sind,

vi) Regenerierung der protonierten Form des Kationenaustauschers durch Behandlung mit einer Säure, und

vii) Verdrängung des polaren Lösungsmittels und der Säure mit einem organischen, apolaren Lösungsmittel.

15. Verfahren gemäss einem der Ansprüche 13 bis 14, dadurch gekennzeichnet, dass das für die Lösung des 2-Aryl-2H-benztriazols und das in den Schritten i), iii), iv) und vii) und gegebenenfalls ii) und v) verwendete organische, apolare Lösungsmittel ein aromatischer Kohlenwasserstoff, insbesondere Xylol oder Toluol, ist, und dass das in den Schritten ii) und v) verwendete polare Lösungsmittel ein einwertiger aliphatischer Alkohol, insbesondere Butanol, ist.